# EUROPEAN PATENT SPECIFICATION

(11) **EP 0 625 887 B1**
(45) Date of publication and mention of the grant of the patent: **14.10.1998**
(21) Application number: 93905044.9
(22) Date of filing: 12.02.1993
(51) Int. Cl.: A61B 17/58, A61L 31/00

(54) **POLYMERIC SCREWS AND COATINGS FOR SURGICAL USES**
POLYMERE SCHRAUBEN UND BESCHICHTUNGEN ZUM CHIRURGISCHEN GEBRAUCH
VIS ET REVETEMENTS POLYMERES A USAGE CHIRURGICAL

(30) Priority: 14.02.1992 US 836674
(43) Date of publication of application: 30.11.1994
(73) Proprietor: SMITH & NEPHEW, INC., Memphis, Tennessee 38116 (US)
(72) Inventor: SMALL, Alan, Attridge, Needham, MA 02194 (US); CARLOZZI, Gerard, Stephen, Weymouth, MA 02190 (US); REGO, Richard, P., Jr., Mansfield, MA 02048 (US); JARRETT, Peter, Kendrick, Southbury, CT 06488 (US)
(74) Representative: Harrison Goddard Foote
(86) International application number: PCT/US93/01284
(87) International publication number: WO 93/15682

(56) References cited:
- EP-A- 0 276 153
- EP-A- 0 451 932
- EP-A- 0 469 441
- WO-A-86/03666
- WO-A-89/09030
- WO-A-90/08510
- US-A- 2 814 296
- US-A- 4 791 929

## Description

### FIELD OF THE INVENTION

This invention relates to polymeric screws and absorbable screw coatings, and more particularly to improved absorbable screws and coated screws for surgical use.

### BACKGROUND OF THE INVENTION

There are a number of surgical procedures in which a fastener such as a screw or nail is inserted into a tissue of a patient. One surgical use involves insertion of an interference screw into a bone tunnel to secure a bone plug in place to attach an end of an anterior cruciate ligament (ACL) replacement. ACL reconstruction procedures and interference screws are disclosed for example in WO 90/08510 U.S. Patent Nos. 5,062,843, 4,950,270 and 4,927,421 and in the following articles: Matthews et al., "Pitfalls in the Use of Interference Screws for Anterior Cruciate Ligament Reconstruction: Brief Report", J. Arthroscopic and Related Surgery 5(3):225-226 (1989) and Kurosaka et al., "A Biomechanical Comparison of Different Surgical Techniques of Graft Fixation in Anterior Cruciate Ligament Reconstruction", Am. J. Sports Medicine 15:225-9 (1987).

Surgeons and patients desire absorbable implants which will be partially or completely absorbed by the body. An absorbable lag screw for fracture fixation and having a rounded head, for example, is disclosed in German Patent DE 3,811,345.

A relatively large amount of torque must be applied to an interference screw during insertion. A screw formed from a bioabsorbable material is likely to have a significantly lower strength, however, and therefore should not be subjected to high torque during insertion. It would be desirable to improve the configuration of the screw or to lower the amount of torque which must be applied to the screw.

Coatings having a low coefficient of friction have been applied to a number of surgical devices including suture needles and hypodermic needles (U.S. Patent No. 2,814,296), and surgical staples (U.S. Patent No. 4,655,222). Typically, most or all of the exterior surface of these devices are coated with a low coefficient of friction material such as Teflon, available from E. I. du Pont de Nemours & Co.

Other uses of a Teflon-type material include incorporation into surgical sutures (U.S. Patent No. 4,362,162), on razor blades (U.S. Patent No. 4,012,551) and on screws to provide self-sealing fasteners (U.S. Patent No. 3,494,243). Other uses include application of absorbable or nonabsorbable plastics (including polytetrafluoroethylene) to staples as disclosed in U.S. Patent No. 4,275,813. Use of Teflon and other resins for resisting corrosion has been disclosed for dental implants in U.S. Patent No. 3,977,081 and for threaded fasteners in U.S. Patent No. 4,835,819.

### SUMMARY OF THE INVENTION

Therefore, it is an object of the present invention to provide improved interference screws formed of a polymeric material which preferably is bioabsorbable.

It is a further object of the invention to provide such screws which can withstand relatively high insertion torques.

A still further object of the invention to provide an absorbable interference screw having increased bite and increased wall thickness to accommodate relatively high edge loads placed on the screw during insertion, and to provide sufficient strength during bioabsorption of the screw until surrounding tissue regenerates sufficiently.

Yet another object of the invention is to provide an absorbable coating for screws to modify the coefficient of friction of the screw, to promote Done ingrowth, or to deliver a medicament or growth factor.

This invention features polymeric interference screws having increased thread thickness and decreased thread depth relative to corresponding metal insertion screws. The increases in screw thicknesses enhance overall rigidity and strength of the absorbable interference Screw. A proximal region of the screw increases gradually in major and/or minor diameter proximally to increase the bite and holding power of the screw as well as to increase wall thickness. A distal region of the screw may define a helical thread having one or more revolutions with a sharp peak at the thread Crest; alternatively, the distal region completely lacks a thread and is a smooth, frustoconical section, and the proximal region is threaded and begins increasing in diameter immediately thereafter.

This invention also features surgical screws having an absorbable coating wherein the base screw is formed of a biocompatible material where the coating is selected to raise or lower the coefficient of friction of the screw, to preferentially biodegrade to promote bone ingrowth, or to serve as a delivery vehicle for a substance such as a pharmaceutical or a growth factor.

### BRIEF DESCRIPTION OF THE DRAWINGS

Other objects, features and advantages will occur from the following description of preferred embodiments and the accompanying drawings, in which:
Fig. 1 is a side view of a conventional metal 5.5mm interference screw;
Fig. 2 is a side view of a novel 5.5mm absorbable interference screw according to the present invention;
Fig. 3 is a side view of a conventional metal 7mm interference screw;
Fig. 4 is a side view of a novel 7mm absorbable interference screw according to the present invention; and
Fig. 5 is a side, partial cross-sectional view of a novel 9mm interference screw which is coated according to the present invention.

### DETAILED DESCRIPTION OF THE PREFERRED EMBODIMENTS

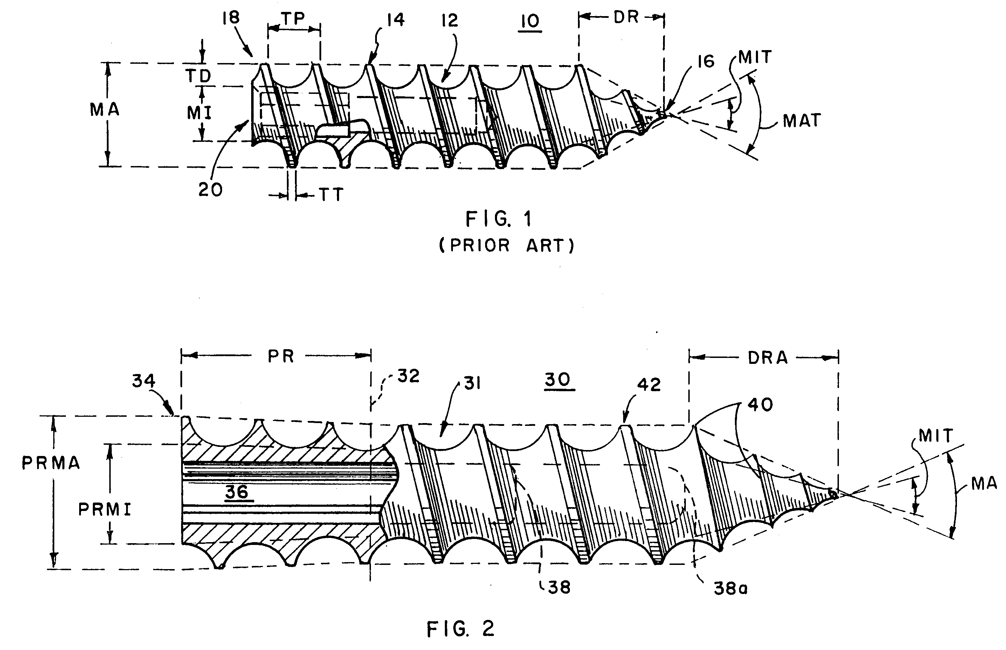

This invention may be accomplished by novel polymeric interference screws such as screws 30, 70 and 90 shown in Figs. 2, 4 and 5 and described below.

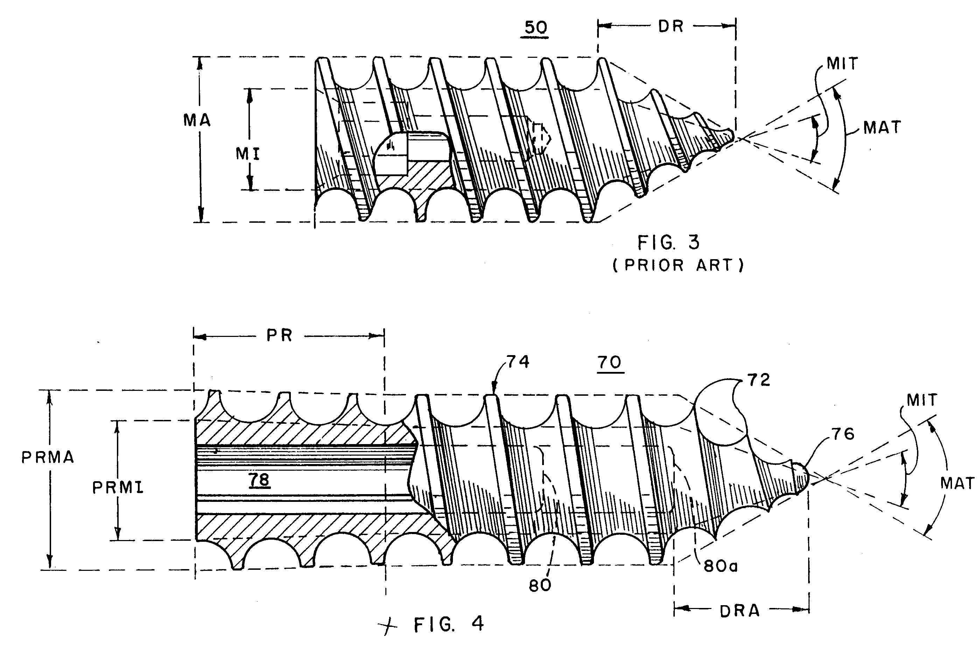

Cannulated and non-cannulated titanium alloy interference screws having a major diameter of 5.5mm for screw 10, Fig. 1, and major diameters of 7.0mm and 9.0mm for screw 50, Fig. 3, are commercially available in lengths of 20mm to 30mm from Acufex Microsurgical, Inc., Mansfield, Massachusetts. Non-cannulated versions are illustrated in Figs. 1 and 3.

Conventional interference screw 10, Fig. 1, includes an elongated body 12 formed of a titanium alloy which defines a helical thread 14 about its exterior surface extending from distal end 16 to proximal end 18. A hexagonal drive socket 20 is defined at the proximal end 18.

Several dimensions applicable to all screws described herein are indicated as thread pitch TP, thread thickness TT, thread depth TD, major diameter MA, and minor diameter MI. Each screw further defines a distal region DR, although the thread thickness in this region is substantially less for polymeric interference screws according to the present invention. Over the proximal remainder of the polymeric screw's helical thread, however, the thread thickness is greater than that of conventional metal screws. For all screws illustrated herein, the helical thread in the distal region DR has a major diameter taper angle of MAT and minor diameter angle taper of MIT; the distal region for polymeric screws is referred to as region DRA.

In general, polymeric interference screws according to the present invention preferably are formed of a bioabsorbable material and have an increased thread thickness TT and a decreased thread depth TD to increase overall rigidity and strength of the absorbable interference screw. This improved construction enables use of bioabsorbable materials which are more brittle and have less strength than metal materials. Thread dimensions are compared in Table I below.

The range of ratios of thread thickness to major diameters as shown in Table I is 0.042 (interference screw 90 at a major diameter of 9mm) to 0.069 (interference screw 30 at 5.5mm) for absorbable interference screws according to the present invention, while the ratios for titanium alloy screws range from 0.028 (screw 50 at a major diameter of 9.0mm) to 0.055 (screw 10 at 5.5mm).

The ratios of thread depth to screw diameter range from 0.178 (screw 90 at 9mm) to 0.185 (screw 70 at 7mm) for the absorbable screws, while the metal screws range from 0.195 (screw 50 at 9.0mm) to 0.229 (screw 10 at 5.5mm).

**TABLE I**

| COMPARISON OF SCREW THREAD DIMENSIONS (INCHES) | | | | |
|---|---|---|---|---|
| Major Diameter | Thread Thickness(TT) | | Thread Depth (TD) | |
| | Metal | Absorbable | Metal | Absorbable |
| .218(5.5mm) | .012 | .015 | .050 | .039 |
| .276(7mm) | .010 | .015 | .069 | .051 |
| .354(9mm) | .010 | .015 | .069 | .063 |

**TABLE II**

| MAXIMUM MAJOR DIAMETER OF PROXIMAL REGION (INCHES) | | | |
|---|---|---|---|
| Major Diameter | Proximal Region Major Diameter (PRMA) By Length | | |
| | 20mm | 25mm | 30mm |
| 5.5mm | .222 | .234 | .246 |
| 7.0mm | .284 | .297 | .311 |
| 9.0mm | .361 | .375 | .389 |

**TABLE III**

| MAXIMUM MINOR DIAMETER OF PROXIMAL REGION (INCHES) | | | | |
|---|---|---|---|---|
| Major Diameter | Minor Diameter | Proximal Region Minor Diameter (PRMI) By Length | | |
| | | 20mm | 25mm | 30mm |
| 5.5mm | .138 | .183 | .195 | .207 |
| 7.0mm | .174 | .233 | .246 | .260 |
| 9.0mm | .228 | .298 | .312 | .326 |

Absorbable interference screw 30 according to the present invention, Fig. 2, has the same major diameter MA of screw 10 up to boundary 32. From boundary 32 to proximal end 34, the elongated body 31 exhibits an increase in major diameter up to a maximum proximal region major diameter of PRMA. Also, there is an increase in minor diameter in proximal region PR up to maximum proximal region minor diameter PRMI.

Different maximum major and minor diameters at the proximal region are provided in Tables II and III for selected lengths of screws 30, 70 and 90 having major diameters of 5.5mm, 7mm and 9mm, respectively. Both the major diameter and minor diameter increase within proximal regions PR at an angle of approximately four degrees to achieve these dimensions. Taper angles of up to ten degrees are desired, with three to five degrees preferred.

The absorbable screw 36 defines a drive socket 36 at its proximal end which extends at least half-way along the length of the elongated body 31 to terminate distally in a socket base 38. In another construction, the socket base continues further to socket base 38a. The drive socket 36 is polygonal when viewed from proximal end 34, preferably the shape disclosed in U.S. Patent No. 3,584,667 (Reiland).

Other features of absorbable screw 30 include a length of proximal region PR of 0.089 to 0.483 inch, a distal minor taper MIT of 32^{°} and a distal major taper MAT of 49^{°}. By comparison, metal screw 10, Fig. 1, has a distal minor taper angle MIT of 32^{°} and a distal major taper angle MAT of 60^{°}. The crests 40 of the helical thread 42 within distal region DRA have a shallower thread and culminate in a peak or edge rather than in a thickened crest as is found in the proximal remainder of the thread 42.

Metal screws have a major diameter MA of 7mm and 9mm and a minor diameter MI of 0.138 inch and 0.216 inch, respectively. Both have a configuration of metal screw 50, Fig. 3. Both sizes have a minor distal taper angle MIT of 36^{°} and a major distal angle taper of 60^{°}.

By comparison, absorbable screw 70, Fig. 4, has a minor distal taper angle of 40^{°} and a major distal taper angle of 60^{°}. At least one revolution of the helical crests 72 of helical thread 74 within distal region DRA culminate in an edge, and the distal tip 76 is rounded. The proximal region PR length is 0.106 to 0.50 inch. Other dimensions are described in Tables I-III. Drive socket 78 terminates distally in a socket base 80 or 80a.

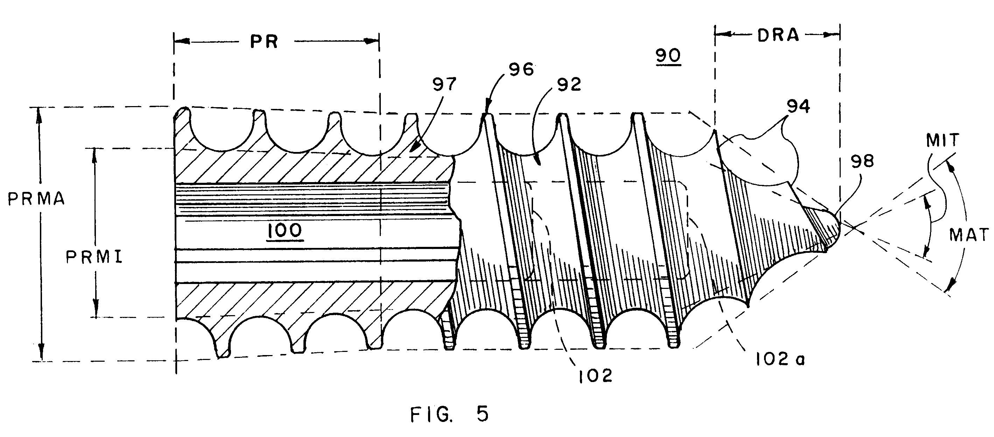

Polymeric interference screw 90, Fig. 5, has its exterior surface covered with a coating 92 as described in more detail below. Dimensions for screw 92 include a minor distal taper angle MIT of 51^{°} and a major distal taper angle MAT of 73^{°}. The proximal region PR has a length of 0.106 to 0.50 inch depending on the overall length of the screw 90. Thread crests 94 of helical thread 96 within distal region DRA have a relatively sharp edge and the elongated screw body 97 terminates in a rounded distal tip 98. Polygonal drive socket 100 terminates distally in a socket base 102 or 102a.

The body of the polymeric screws according to the present invention can he formed of a non-absorbable polymer such as Delrin available from Du Pont or of a bioabsorbable material such as polylactic acid (lactide), polyglycolic acid (glycolide) disclosed in U.S. Patent No. 3,739,773 (Schmitt et al.), or copolymers disclosed in U.S. Patent Nos. 4,300,565 (Rosensaft et al.) and 4,429,080 (Casey et al.), all of which are incorporated herein by reference. A combination of absorbable and non-absorbable materials to form a partially absorbable implant can also be utilized. A polymer such as lactide is preferred for its slower absorption rate and therefore longer retention of structural integrity. Retention of at least fifty percent strength twelve weeks after implantation is preferred for an interference screw used to secure a ligament graft in position.

The absorbable coating 92 preferably has a low coefficient of friction such as polycaprolate. An acceptable polycaprolate copolymer is a random copolymer of 85 weight percent epsilon-caprolactone and 15 weight percent glycolide. Other suitable coatings are disclosed in U.S. Patent No. 4,788,979 (Jarrett et al.), which is incorporated herein by reference. In addition to reducing insertion torque, the absorbable coating can contain a pharmaceutical, a growth factor, or other compound. Additionally, the coating can have a selected absorption property to disappear within a desired period to enhance bone ingrowth and attachment to the screw.

In addition to application on polymeric interference screws, an absorbable coating can also be placed according to the invention on a metal screw such as screws 10 and 50, Figs. 1 and 3. The above-described advantages of an absorbable coating apply also to these non-polymeric, nonabsorbable surgical screws. Such coated screws are particularly useful for fracture fixation, and can be cannulated or non-cannulated.

As a coating over an absorbable screw body, the absorbable coating can serve as a barrier to body fluids to affect the rate of absorption of the main screw body. An absorbable coating according to the invention has a different composition than the screw body to provide one or more different selected properties such as a different coefficient of friction or rate of absorption.

One example of a metal screw coated according to the present invention is provided by five titanium alloy screws which were dipped in a solution of 10g polycaprolate copolymer (85 weight percent caprolactone, 15 weight percent glycolide) in 80ml of acetone to form a 12.75% solution weight/volume. The screws were alternately dipped and allowed to dry for a total of seven (7) times.

A total of ten screws were tested, five uncoated 7mm by 25mm non-cannulated interference screws supplied by Acufex Microsurgical, Inc. and having the configuration shown in Fig. 3, and five identical screws dipcoated with polycaprolate as described above. One of each screw was inserted into the middle intra-condylar region of a bovine bone, two each in the posterior condylar region, and two each in the anterior condylar region. The results are provided below in Table IV.

**TABLE IV**

| COATED VERSUS UNCOATED SCREW INSERTION TORQUES (IN.LBS) | | |
|---|---|---|
| Bone Region | Coated | Uncoated |
| Middle Condyle | 11.6 | 19.8 |
| Posterior Condyle | 24.6 | 28.6 |
| | 19.0 | 29.8 |
| Anterior Condyle | 22.0 | 22.8 |
| | 21.6 | 25.4 |

The average coated screw required 19.8 in. lb. of torque whereas the average uncoated screw required 25.3 in. lb. An average reduction in torque of 22% was therefor provided by the absorbable coating.

Although specific features of the invention are shown in some drawings and not in others, this is for convenience only as each feature may be combined with any or all of the other features in accordance with the invention.

Other embodiments will occur to those skilled in the art within the scope of the following claims.

## Claims

1. An interference screw insertable into a tunnel drilled in a bone to secure a graft anchor plug in place, comprising an elongated, substantially cylindrical screw body having a distal end and a proximal end, said proximal end defining means for engaging a driver and lacking a head member, said elongated body formed of a polymeric material, said elongated body defining a helical thread about its exterior and having a body major diameter and a body minor diameter, and said elongated body further defining a distal region which decreases in diameter advancing distally and terminates in said distal end, and defining a proximal region which, advancing proximally toward said proximal end, increases to at least one of (a) a proximal region major diameter greater than said body major diameter, and (b) a proximal region minor diameter greater than said body minor diameter.

2. The interference screw of Claim 1 in which said elongated body is formed of a bioabsorbable material and said helical thread extends over said distal region with a substantially narrower thread width, for at least one revolution of said thread, than the thread width of the proximal remainder of said helical thread.

3. The interference screw of Claim 2 in which said elongated body terminates in a substantially rounded distal tip at its distal end.

4. The interference screw of Claim 1 in which said elongated body is formed of a bioabsorbable material and further including a bioabsorbable coating disposed about the exterior surface of said elongated body, said coating having a coefficient of friction which is lower than that of the exterior surface of said elongated body.

5. The interference screw of Claim 4 in which said coating is a polycaprolate-type material.

6. The interference screw of Claim 5 in which said polycaprolate-type material is a random copolymer of 85 weight percent epsilon-caprolactone and 15 weight percent glycolide.

7. The interference screw of Claim 1 in which said proximal region major diameter tapers outwardly at an angle of up to ten degrees.

8. The interference screw of Claim 1 in which said proximal region major diameter tapers outwardly at an angle of about three to five degrees.

## Patentansprüche

1. Interferenzschraube, die zum Befestigen eines Dübels zur Implantatverankerung an der Verwendungsstelle in einen Tunnel einsetzbar ist, der in einen Knochen gebohrt wurde, umfassend einen länglichen, im wesentlichen zylindrischen Schraubenkörper mit einem spitzenseitigen Ende und einem der Spitze abgewandten Ende, wobei durch das der Spitze abgewandte Ende Mittel zum Eingriff eines Eindrehwerkzeuges definiert werden und ihm ein Kopfteil fehlt, der längliche Körper aus einem polymeren Material gebildet ist, durch den länglichen Körper ein Schraubengewinde um sein Äußeres definiert wird und er einen größeren Körperdurchmesser und einen kleineren Körperdurchmesser aufweist und durch den länglichen Körper weiter ein spitzenseitiger Bereich, dessen Durchmesser sich zur Spitzenseite hin fortschreitend verringert und der im spitzenseitigen Ende endet, und ein der Spitze abgewandter Bereich definiert wird, der sich zum der Spitze abgewandten Ende fortschreitend auf wenigstens einen von (a) einem größeren Durchmesser des der Spitze abgewandten Bereiches, der größer als der größere Körperdurchmesser ist, und (b) einem kleineren Durchmesser des der Spitze abgewandten Bereiches, der größer als der kleinere Körperdurchmesser ist, erhöht.

2. Interferenzschraube von Anspruch 1, wobei der ländliche Körper aus einem bioabsorbierbaren Material gebildet ist und das Schraubengewinde sich über den spitzenseitigen Bereich mit einer wesentlich schmaleren Gewindebreite als der Gewindebreite des der Spitze abgewandten übrigen Schraubengewindes über wenigstens einen Gewindegang erstreckt.

3. Interferenzschraube von Anspruch 2, wobei der längliche Körper an seinem spitzenseitigen Ende in einer im wesentlichen runden spitzenseitigen Spitze endet.

4. Interferenzschraube von Anspruch 1, wobei der längliche Körper aus einem bioabsorbierbaren Material gebildet ist und weiter einen bioabsorbierbaren Überzug einschließt, der über der äußeren Oberfläche des länglichen Körpers angeordnet ist, wobei der Überzug einen Reibungskoeffizienten aufweist, der niedriger als der der äußeren Oberfläche des länglichen Körpers ist.

5. Interferenzschraube von Anspruch 4, wobei der Überzug ein Material vom Polycaprolattyp ist.

6. Interferenzschraube von Anspruch 5, wobei das Material vom Polycaprolattyp ein Random-Copolymer aus 85 Gew.-% δ-Caprolacton und 15 Gew.-% Glycolid ist.

7. Interferenzschraube von Anspruch 1, wobei sich der größere Durchmesser des der Spitze abgewandten Bereiches mit einem Winkel von bis zu zehn Grad nach außen verjüngt.

8. Interferenzschraube von Anspruch 1, wobei sich der größere Durchmesser des der Spitze abgewandten Bereiches mit einem Winkel von etwa drei bis fünf Grad nach außen verjüngt.

## Revendications

1. Vis à interférence pouvant être insérée dans un canal percé dans un os pour fixer en position une broche d'ancrage d'un greffon, comprenant un corps de vis allongé sensiblement cylindrique possédant une extrémité distale et une extrémité proximale, ladite extrémité proximale définissant des moyens permettant l'engagement d'un tournevis et ne comportant pas d'élément de tête, ledit corps allongé étant formé d'un matériau polymère, ledit corps allongé définissant un filetage hélicoïdal sur sa face extérieure et possédant un grand diamètre et un petit diamètre, et ledit corps allongé définissant en outre une région distale, dont le diamètre diminue lorsqu'on s'avance vers le côté distal, et se termine dans ladite extrémité distale, et définissant une région proximale qui, lorsqu'on avance sur le côté proximal en direction de l'extrémité proximale, augmente jusqu'à au moins l'un de (a) un grand diamètre de la région proximale supérieur audit grand diamètre du corps, et (b) un petit diamètre de la région proximale supérieur audit petit diamètre du corps.

2. Vis à interférence selon la revendication 1, dans laquelle ledit corps allongé est formé d'un matériau bioabsorbable et ledit filetage hélicoïdal s'étend sur ladite région distale avec une largeur de filetage nettement plus faible, sur au moins une rotation de ladite vis, que la largeur du filetage du reste proximal dudit filetage hélicoïdal.

3. Vis à interférence selon la revendication 2, dans laquelle ledit corps allongé se termine par une pointe distale essentiellement arrondie au niveau de son extrémité distale.

4. Vis à interférence selon la revendication 1, dans laquelle ledit corps allongé est formé d'un matériau bioabsorbable, et comprenant en outre un revêtement bioabsorbable disposé autour de la surface extérieure dudit corps allongé, ledit revêtement possédant un coefficient de frottement qui est inférieur à celui de la surface extérieure dudit corps allongé.

5. Vis à interférence selon la revendication 4, dans laquelle ledit revêtement est un matériau du type polycaprolate.

6. Vis à interférence selon la revendication 5, dans laquelle ledit matériau du type polycaprolate est un copolymère aléatoire formé de quatre-vingt-cinq pour-cent de epsilon-caprolactone et de 15 pour-cent en poids de glycolide.

7. Vis à interférence selon la revendication 1, dans laquelle la partie de grand diamètre de la région proximale se rétrécit vers l'extérieur sous un angle atteignant jusqu'à dix degrés.

8. Vis à interférence selon la revendication 1, dans laquelle ladite partie de grand diamètre de la région proximale se rétrécit vers l'extérieur sous un angle d'environ trois à cinq degrés.
